# FASCICULE DE BREVET EUROPEEN

(11) **EP 2 018 425 B1**
(45) Date de publication et mention de la délivrance du brevet: **04.09.2013**
(21) Numéro de dépôt: 07766074.4
(22) Date de dépôt: 18.05.2007
(51) Int. Cl.: C12N 15/10, C07H 1/08, C12Q 1/68, C12Q 1/04, C12Q 1/24

(54) **PROCEDE D'EXTRACTION DES ACIDES DESOXYRIBONUCLEIQUES (ADN) DE MICROORGANISMES EVENTUELLEMENT PRESENTS DANS UN ECHANTILLON SANGUIN**
VERFAHREN ZUR EXTRAKTION VON DESOXYRIBONUKLEINSÄUREN (RNA) AUS IN EINER BLUTPROBE MÖGLICHERWEISE VORHANDENEN MIKROORGANISMEN
METHOD FOR EXTRACTING DEOXYRIBONUCLEIC ACIDS (DNA) FROM MICROORGANISMS POSSIBLY PRESENT IN A BLOOD SAMPLE

(30) Priorité: 19.05.2006 FR 0604531
(43) Date de publication de la demande: 28.01.2009
(73) Titulaire: GeneOhm Sciences, Inc., San Diego CA 92121 (US)
(72) Inventeur: HERMET, Jean-Pierre, F-92100 Boulogne-Billancourt (FR); BESSON-FAURE, Isabelle, F-91330 Yerres (FR); HOULLE-DECLOMESNIL, Anne-Elodie, F-14740 Bretteville L'Orgueilleuse (FR)
(74) Mandataire: Novagraaf Technologies
(86) Numéro de dépôt international: PCT/FR2007/051300
(87) Numéro de publication internationale: WO 2007/135332

(56) Documents cités:
- WO-A-03/025207
- RIBAULT S ET AL: "Detection of bacteria in red blood cell concentrates by the Scansystem method." JOURNAL OF CLINICAL MICROBIOLOGY. MAY 2005, vol. 43, no. 5, mai 2005 (2005-05), pages 2251-2255, XP002402268 ISSN: 0095-1137
- RIBAULT S ET AL: "Rapid screening method for detection of bacteria in platelet concentrates." JOURNAL OF CLINICAL MICROBIOLOGY. MAY 2004, vol. 42, no. 5, mai 2004 (2004-05), pages 1903-1908, XP002402269 ISSN: 0095-1137
- LEW A E ET AL: "DETECTION OF PSEUDOMONAS PSEUDOMALLEI BY PCR AND HYBRIDIZATION" JOURNAL OF CLINICAL MICROBIOLOGY, WASHINGTON, DC, US, vol. 32, no. 5, mai 1994 (1994-05), pages 1326-1332, XP000921285 ISSN: 0095-1137
- MCDONALD C P ET AL: "Use of a solid-phase fluorescent cytometric technique for the detection of bacteria in platelet concentrates." TRANSFUSION MEDICINE (OXFORD, ENGLAND) JUN 2005, vol. 15, no. 3, juin 2005 (2005-06), pages 175-183, XP002402270 ISSN: 0958-7578
- JACOBS MICHAEL R ET AL: "Evaluation of the Scansystem method for detection of bacterially contaminated platelets." TRANSFUSION. FEB 2005, vol. 45, no. 2, février 2005 (2005-02), pages 265-269, XP002402271 ISSN: 0041-1132

## Description

La présente invention se rapporte au domaine de la détection et de l'identification de microorganismes éventuellement présents dans un échantillon sanguin. Elle concerne tout particulièrement un procédé d'extraction des acides désoxyribonucléiques (ADN) de microorganismes éventuellement présents dans un échantillon sanguin en vue de leur identification.

La mise au point de méthodes simples et rapides permettant la détection et/ou l'identification et/ou la détermination du taux de microorganismes éventuellement présents dans un échantillon sanguin est un enjeu majeur notamment dans le domaine de la santé.

Actuellement, les méthodes de biologie moléculaire et particulièrement les techniques de réaction en chaîne par polymérase (PCR) sont en pleine expansion.

En effet, ces techniques présentent une grande sensibilité et spécificité pour l'identification et/ou la quantification des microorganismes. Toutefois, l'application de ces méthodes de biologie moléculaire et particulièrement les techniques de réaction en chaîne par polymérase (PCR) à des échantillons sanguins présente des difficultés, notamment en termes de sensibilité et de réalisation.

En effet, les échantillons sanguins comprennent des agents inhibiteurs des techniques de biologie moléculaire et en particulier des techniques de réaction en chaîne par polymérase (PCR).

Ces agents inhibiteurs peuvent interférer avec les procédés d'extraction d'ADN et/ou dégrader les acides désoxyribonucléiques (ADN) des cellules et/ou inhiber l'activité des enzymes, notamment des polymérases utilisées dans différentes techniques de biologie moléculaire.

À titre d'exemples de tels agents inhibiteurs en particulier des techniques de réaction en chaîne par polymérase (PCR), notamment présents dans le sang total, on peut citer les ions calcium, l'hémoglobine, la lactoferrine, l'hémine, l'urée et l'héparine du sang (Al-Soud, W.A. and Radstrom, P. 2001. Purification and characterization of PCR-inhibitory components in blood cells. J Clin Microbiol 39:2:485-493). Il subsiste donc un besoin pour des procédés d'extraction d'ADN de microorganismes présents dans un échantillon sanguin, en vue de leur détection et/ou de leur identification.

Les inventeurs ont découvert un procédé particulier permettant de résoudre en tout ou partie les problèmes évoqués ci-dessus.

Selon un premier aspect, l'invention a pour objet un procédé d'extraction d'ADN de microorganismes éventuellement présents dans un échantillon sanguin comprenant les étapes suivantes :
i) la filtration d'un échantillon sanguin au travers d'une membrane de filtration dont les pores présentent un diamètre allant de 0,01 µm à 50 µm, en particulier de 0,1 µm à 10 µm et tout particulièrement de 0,2 µm à 1 µm ;
ii) le lavage de ladite membrane de filtration, dans lequel ledit lavage lyse les globules rouges de l'échantillon sanguin; et
iii) l'extraction des acides désoxyribonucléiques des microorganismes éventuellement présents sur ladite membrane de filtration.

On entend par « échantillon sanguin » au sens de la présente invention un échantillon de sang total ou d'hémoculture, qui a éventuellement été traité en vue de réduire le taux et/ou d'éliminer les globules rouges et/ou les plaquettes présents dans ledit échantillon.

L'étape de traitement d'un échantillon sanguin en vue de réduire le taux et/ou d'éliminer les globules rouges et/ou les plaquettes présents dans ledit échantillon peut être réalisée selon des techniques bien connues de l'Homme du Métier.

À titre d'exemples de telles techniques, on peut citer celles décrites dans la Demande PCT WO 03/025207:
- la technique d'agrégation plaquettaire à l'aide d'agents d'agrégation tels que des anticorps spécifiques d'un antigène plaquettaire, suivie de la filtration de l'échantillon traité ;
- la technique d'agglutination des globules rouges à l'aide d'agents d'agrégation tels que les lectines suivie de la filtration de l'échantillon traité.

Les échantillons sanguins de sang total peuvent être obtenus selon des techniques bien connues de l'Homme du Métier à l'aide par exemple d'une aiguille munie d'une seringue introduite en particulier dans une veine de l'avant-bras ou du pli du coude d'un individu. Un échantillon de 1 à 10 mL de sang prélevé en particulier sur anticoagulant, notamment EDTA, citrate de sodium ou héparine, obtenu chez un sujet humain ou animal peut être suffisant pour mettre en oeuvre le procédé selon la présente invention.

Les échantillons sanguins d'hémoculture peuvent être obtenus après ensemencement du sang total prélevé chez un sujet humain ou animal sur des milieux de culture appropriés au développement des microorganismes.

Une membrane de filtration adaptée pour le procédé selon l'invention peut être identifiée simplement par l'Homme du Métier au regard de ses connaissances générales.

En particulier, la membrane de filtration selon l'invention peut être choisie dans le groupe comprenant les membranes en fluoride de polyvinylidène, en polyester, en nylon, en polypropylène, en polycarbonate et en polyethersulfone, en particulier en fluoride de polyvinylidène.

De préférence, ladite membrane de filtration n'est pas à base de cellulose.

L'étape i) de filtration du procédé selon l'invention peut être effectuée à l'aide de dispositifs et de supports de filtre bien connus de l'Homme du Métier, notamment un support tel que décrit dans la Demande de Brevet US 2004/0208796.

À l'étape ii), on entend par « lavage», une étape permettant de réduire le taux des impuretés retenues sur la membrane de filtration tout en permettant à au moins une partie des microorganismes d'être maintenus sur ladite membrane.

Les impuretés peuvent être notamment des agents inhibiteurs des techniques de biologie moléculaire (Wilson,J.G. 1997. Inhibition and Facilitation of Nucleic Acid Amplification. Appl Environ Microbiol 63:10:3741-3751), notamment :
- des protéines plasmatiques, les immunoglobulines G (Al-Soud, W.A., Jonsson, L.J., and Radstrom, P. 2000. Identification and characterization of immunoglobulin G in blood as a major inhibitor of diagnostic PCR. J Clin Microbiol 38:1:345-350);
- des enzymes (protéases) ;
- des polyamines ;
- du polyanéthole sulfonate de sodium (SPS) ;
- des anticoagulants sanguins comme l'héparine (Satsangi, J., Jewell, D.P., Welsh, K., Bunce, M., and Bell, J.I. 1994. Effect of heparin on polymerase chain reaction. Lancet 343:8911:1509-1510) et l'acide éthylènediaminetétraacétique (EDTA) (Al-Soud, W.A. and Radstrom, P. 2001. Purification and characterization of PCR-inhibitory components in blood cells. J Clin Microbiol 39:2:485-493);
- l'hémoglobine, l'hémine (Akane, A., Matsubara, K., Nakamura, H., Tahahashi, S., and Kimura, K. 1994. Identification of the heme compound copurified with deoxyribonucleic acid (DNA) from bloodstains, a major inhibitor of polymerase chain reaction (PCR) amplification. J Forensic Sci 39:2:362-372), la bilirubine, les phénols ;
- des détergents tels que le sodium dodecyl sulfate, le tryton X100 ; et
- les acides biliaires.

Ladite étape ii) permet de lyser, en particulier par un choc hypotonique, les globules rouges de l'échantillon sanguin et en particulier d'éliminer leur contenu, notamment l'hémoglobine contenue dans ces globules rouges.

L'Homme du Métier pourra déterminer simplement le volume de solution de lavage utilisée à l'étape ii) au regard de ses connaissances générales.

Le volume de solution de lavage peut correspondre à un volume compris entre ¼ et 20, en particulier entre ½ et 10 et tout particulièrement entre 1 et 5 fois le volume de l'échantillon sanguin filtré à l'étape i).

Par exemple, lorsque l'échantillon sanguin filtré à l'étape i) a un volume allant de 0,2 à 5 mL et tout particulièrement de 0,5 à 1 mL, alors l'étape ii) de lavage peut être effectuée avec un volume de solution de lavage allant de 1 à 10 mL, en particulier de 3 à 5 mL et tout particulièrement de 3 mL.

À titre d'exemple encore, lorsque l'échantillon sanguin filtré à l'étape i) a un volume allant de 5 à 100 mL, tout particulièrement de 5 à 20 mL, alors l'étape ii) de lavage peut être effectuée avec un volume de solution de lavage allant de 5 à 50 mL, en particulier de 8 à 20 mL et tout particulièrement de 10 mL.

La solution de lavage adaptée pour le procédé selon l'invention peut être identifiée simplement par l'Homme du Métier au regard de ses connaissances générales.

La solution de lavage peut être choisie notamment parmi des solutions aqueuses, en particulier de l'eau et tout particulièrement de l'eau osmosée, préférentiellement stérilisée par filtration.

Ladite stérilisation de l'eau par filtration peut être réalisée notamment en utilisant une membrane de filtration dont les pores peuvent présenter un diamètre d'environ 0,22 µm.

À titre d'exemples de solutions de lavage utilisables pour l'étape ii) de lavage selon l'invention, on peut citer l'eau pure pour biologie moléculaire disponible chez Eppendorf avec notamment la référence 0032.006.159, l'eau pure issue des systèmes connus de purification avec notamment le système Purelab® de chez ELGA Labwater, ou le système Milli-Q® de chez Millipore, ou le système Infinity UV/UF® de chez Werner.

Lorsque le procédé selon l'invention utilise un dispositif et/ou un support de filtre, l'étape ii) de lavage peut être effectuée notamment par passage de la solution de lavage sur ladite membrane de filtration, en laissant en place la membrane de filtration dans le dispositif et/ou sur le support de filtre utilisé à l'étape i).

L'étape d'extraction des acides désoxyribonucléiques de microorganismes éventuellement présents sur ladite membrane de filtration peut être effectuée selon des techniques bien connues de l'Homme du Métier telles que des méthodes de lyse physique notamment thermique ou par sonication, des méthodes de lyse chimique, notamment décrites dans le manuel de Sambrook J., Fritsch E.F. et Maniatis T. (Molecular cloning : a laboratory manuel, 2nd ed. Cold Spring Harbor Laboratory Press, Cold Spring, N.Y., 1989

On entend par « microorganisme » un organisme vivant appartenant à l'un des trois règnes suivants, celui des monères, des protistes et des protozoaires. Les microorganismes présentent une structure cellulaire eucaryote ou procaryote, ou acaryote, une taille microscopique ou ultramicroscopique et sont unicellulaires. À titre d'exemples de microorganismes, on peut citer :
- les bactéries telles que Escherichia coli, klebsielle, shigelle, streptocoques, staphylocoques, enterocoques, proteus, enterobacter, serratia, pseudomonas, bacillus, corynebactéries, listeria, acinetobacter, cryptocoques, bartonella, mycobactéries ; et
- les champignons tels que candida, aspergillus.

Tout particulièrement, le procédé selon l'invention comprend en outre l'étape suivante :
iv) l'identification des microorganismes notamment des bactéries, des virus, des protozoaires et/ou des champignons éventuellement présents dans ledit échantillon sanguin.

On entend par « identification », la détermination de l'espèce d'un microorganisme.

L'identification des microorganismes peut être effectuée à partir des acides désoxyribonucléiques extraits desdits microorganismes par des techniques de biologie moléculaire bien connues de l'Homme du Métier.

En particulier, l'étape iv) comprend l'utilisation d'une technique utilisant une activité de type polymérase choisie dans le groupe comprenant la réaction en chaîne par polymérase en point final, la réaction en chaîne par polymérase multiplex, la réaction en chaîne par polymérase qualitative, la réaction en chaîne par polymérase semi-quantitative , la réaction en chaîne par polymérase quantitative.

Des appareils de PCR en point final disponibles en particulier chez Applied Biosystems sous la dénomination « ABI PRISM® », chez Roche Diagnostics sous la dénomination « COBAS Amplicor® » peuvent être utilisés dans le procédé selon l'invention.

Des appareils de PCR en temps réel disponibles en particulier chez Applied Biosystems sous la dénomination « 7500 Real-Time PCR System® », chez Roche Diagnostics sous la dénomination « COBAS Taqman® » et chez Genesystems sous la dénomination « GeneDisc cycler® » peuvent être utilisés dans le procédé selon l'invention.

Des kits de PCR en temps réel avec des couples d'amorces et sondes spécifiques d'un microorganisme disponibles chez Roche avec le « Lightcycler SeptiFast kit® » sous les références 04469046001 ou 04488814001); chez Biotage avec les kits « microbial species determination and résistance » sous les références 8, 7 et 12; chez BAG (Biologish Analysensystem GMbH) avec les kits « Hyplex StaphyloResist® » sous la référence 3801, « Hyplex StaphyloResist » plus sous la référence 3809, « Hyplex EnteroResist® » sous la référence 3802 peuvent être utilisés dans le procédé selon l'invention Par exemple, le kit de PCR en temps réel disponible chez Argène avec la référence 69-002 est utilisable pour identifier et déterminer le taux de virus d'Epstein-Barr (EBV) présent dans un échantillon sanguin.

Selon un mode de réalisation particulier, le procédé selon l'invention comprend en outre l'étape suivante :
v) l'identification d'au moins un gène de résistance à un antibiotique chez au moins un microorganisme éventuellement présent dans ledit échantillon sanguin.

L'identification d'un gène de résistance à un antibiotique à partir des acides désoxyribonucléiques extraits des microorganismes peut être effectuée par des techniques de biologie moléculaire bien connues de l'Homme du Métier.

En particulier, l'étape v) comprend l'utilisation d'une technique de réaction en chaîne par polymérase avec notamment des couples d'amorces et éventuellement de sondes spécifiques d'au moins un gène de résistance à un antibiotique.

Selon un autre mode de réalisation particulier, le procédé selon l'invention comprend en outre l'étape suivante :
vi) la détermination du taux de microorganismes, notamment des bactéries, des virus, des protozoaires et/ou des champignons éventuellement présents dans ledit échantillon sanguin.

On entend par « taux de microorganismes », la quantité de microorganismes présents dans l'échantillon sanguin sur lequel le procédé selon l'invention est mise en oeuvre.

La détermination du taux de microorganismes éventuellement présents dans ledit échantillon sanguin peut être effectuée par des techniques bien connues de l'Homme du Métier.

À titre d'exemple, cette détermination peut s'effectuer en comparant les résultats obtenus à l'étape iv) d'identification des microorganismes avec les résultats obtenus avec des témoins positifs correspondant à des dilutions déterminées desdits microorganismes.

En particulier, l'étape vi) comprend l'utilisation de la technique de la réaction en chaîne par polymérase en temps réel.

Avantageusement au moins une des étapes iv), v), vi) du procédé selon l'invention est réalisée dans un milieu adapté à au moins une technique de biologie moléculaire, comprenant un extrait des acides désoxyribonucléiques des microorganismes obtenu à l'étape iii).

On entend par « milieu adapté à au moins une technique de biologie moléculaire », un milieu comprenant des agents permettant d'augmenter le rendement et/ou la sensibilité et/ou la spécificité de ces techniques, notamment des réactions en chaîne par polymérase.

De tels milieux sont bien connus de l'Homme du Métier, tels que décrits dans la littérature (Wilson, J.G. 1997. Inhibition and Facilitation of Nucleic Acid Amplification. Appl Environ Microbiol 63:10:3741-3751).

À titre d'exemple de milieux adaptés aux techniques de PCR, on peut citer les milieux comprenant notamment de l'albumine sérique bovine (Akane, A., Matsubara, K., Nakamura, H., Tahahashi, S., and Kimura, K. 1994. Identification of the heme compound copurified with deoxyribonucleic acid (DNA) from bloodstains, a major inhibitor of polymerase chain reaction (PCR) amplification. J Forensic Sci 39:2:362-372), du glycérol, des ions magnésium (Satsangi, J., Jewell, D.P., Welsh, K., Bunce, M., and Bell, J.I. 1994. Effect of heparin on polymerase chain reaction. Lancet 343:8911:1509-1510), de la triméthyl glycine, du diméthylsulfoxide, du polyéthylène glycol, du Tween 20®, du Tween 40®, du Tween 80®, du DMSO, du Triton X-100®, du Triton X-114®, de la bétaine monohydrate, de la bétaine triméthylglycine, du PEG 35000, du PEG 400, du PEG 6000 et de l'acétamide.

Selon un mode de réalisation particulier, le procédé selon l'invention, comprend préalablement à l'étape i), les étapes suivantes :
a) l'addition au sang total ou à l'hémoculture d'une solution d'agglutination des globules rouges et/ou d'une solution d'agrégation plaquettaire ; et
b) la filtration de la préparation obtenue à l'étape a) au travers d'un filtre dont les pores présentent un diamètre allant de 2 µM à 50 µM, en particulier allant de 10 µM à 25 µM et tout particulièrement de 17 µm.

En particulier, ladite solution d'agglutination des globules rouges comprend au moins un agent d'agglutination choisi dans le groupe comprenant les lectines, le polyéthylènimine, la polyvinylpyrrolidone, les gélatines, les dextranes et les polyéthylènes glycols, en particulier les lectines.

Tout particulièrement, les lectines sont choisies dans le groupe comprenant les lectines de *lens culinaris, Phaseolus vulgaris, Vicia sativa, Vicia faba* et de *Erythrina corallodendron.*

L'agent d'agglutination, notamment la lectine, peut être présent dans la solution d'agglutination à une concentration allant de 10 µg/ml à 200 µg/ml, en particulier de 15 µg/ml à 100 µg/ml et tout particulièrement de 20 µg/ml à 30 µg/ml.

En particulier, ladite solution d'agrégation plaquettaire comprend au moins un agent d'agrégation plaquettaire choisi dans le groupe comprenant les anticorps spécifiques d'un antigène plaquettaire, la thrombine, la trypsine, le collagène, le thromboxane A2, le facteur d'activation plaquettaire, l'adrénaline, l'acide arachidonique, la sérotonine et l'épinéphrine, en particulier les anticorps spécifiques d'un antigène plaquettaire et le collagène.

Les anticorps spécifiques d'un antigène plaquettaire peuvent être présents dans la solution d'agrégation plaquettaire à une concentration allant de 0,5 µg/ml à 100 µg/ml, en particulier de 1 µg/ml à 60 µg/ml et tout particulièrement de 5 µg/ml à 45 µg/ml.

Le collagène peut être présent dans la solution d'agrégation plaquettaire à une concentration allant de 0,05 µg/ml à 50 µg/ml, en particulier de 1 µg/ml à 20 µg/ml

D'autres avantages et caractéristiques de l'invention apparaîtront au regard des figures et des exemples qui suivent.

Les figures et les exemples qui suivent sont donnés à titre illustratif et non limitatif :

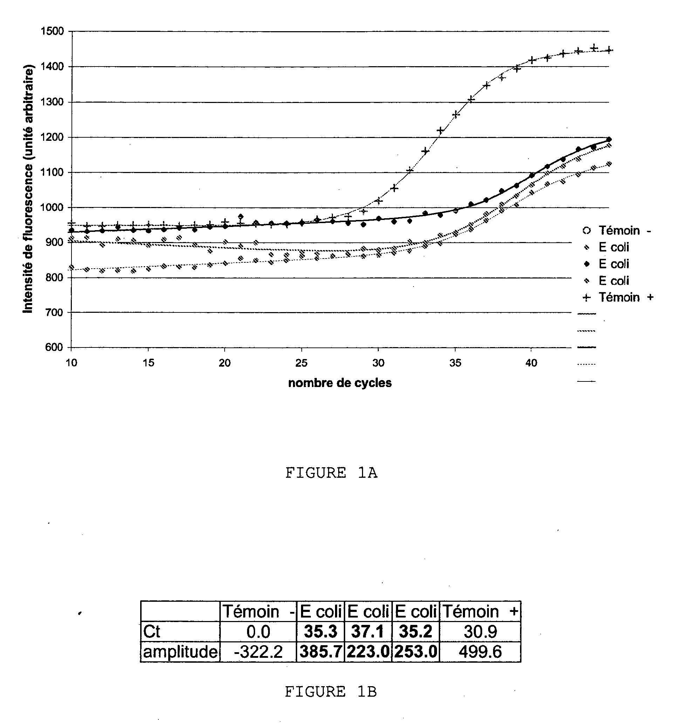

- la figure 1 (A et B) illustre l'identification et la quantification d'Escherichia Coli présents dans des échantillons sanguins de sang total. La figure 1A illustre sous forme de courbe les résultats des réactions en chaîne par polymérase en temps réel utilisant une sonde fluorescente et un couple d'amorces spécifiques d'Escherichia Coli. La figure 1B illustre sous forme d'un tableau le cycle seuil et l'amplitude des réactions en chaîne par polymérase en temps réel.

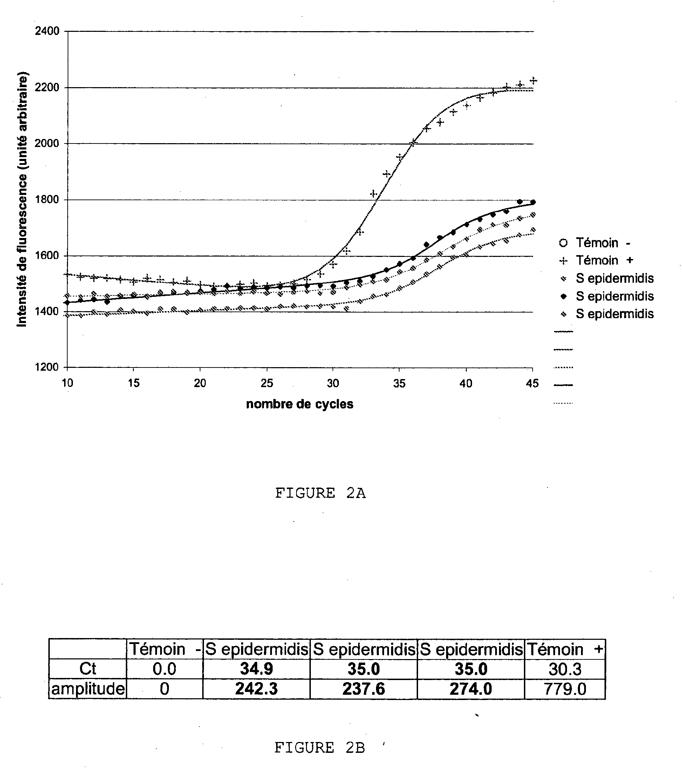

- la figure 2 (A et B) illustre l'identification et la quantification de Staphylococcus epidermis présents dans des échantillons sanguins de sang total. La figure 2A illustre sous forme de courbe les résultats des réactions en chaîne par polymérase en temps réel utilisant une sonde fluorescente et un couple d'amorces spécifiques de Staphylococcus epidermis. La figure 2B illustre sous forme d'un tableau le cycle seuil et l'amplitude des réactions en chaîne par polymérase en temps réel.

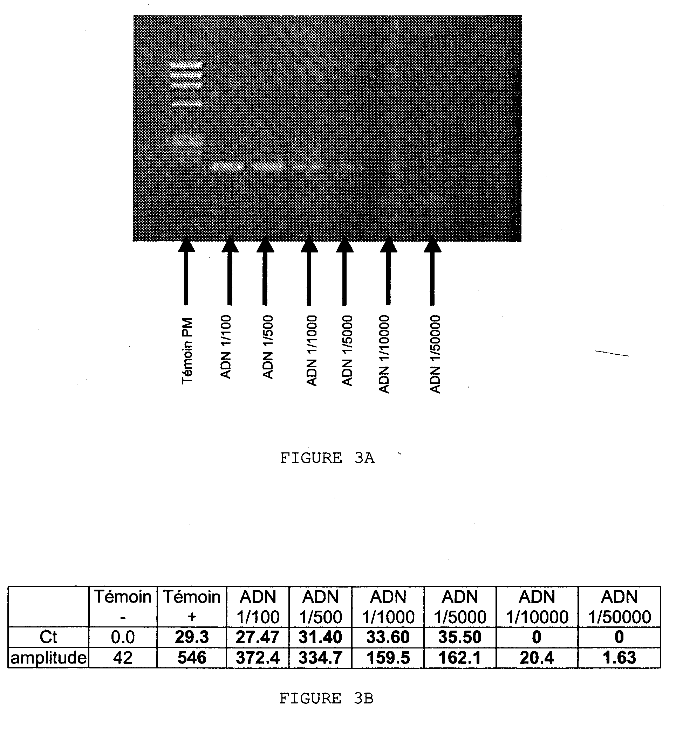

- la figure 3A montre une photo d'un gel d'agarose après réaction en chaîne par polymérase (PCR) en point final et migration en utilisant une sonde fluorescente et un couple d'amorces spécifiques d'Escherichia coli.
- la figure 3B montre les résultats des réactions en chaîne par polymérase (PCR) quantitatives en temps réel en utilisant une sonde fluorescente et un couple d'amorces spécifiques d'Escherichia coli.

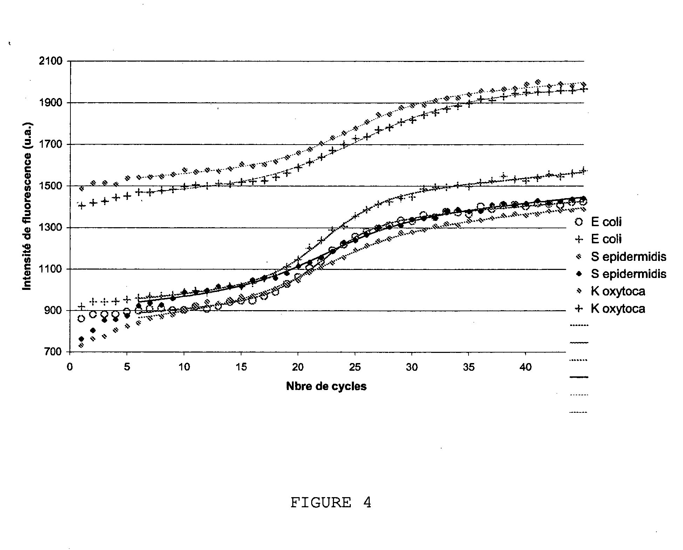

- la figure 4 illustre sous la forme de courbes l'identification et la quantification d'Escherichia coli, de Staphylococcus epidermidis et de Klebsiella oxytoca présents dans des échantillons sanguins d'hémoculture. La figure 4 illustre sous forme de courbe les résultats des réactions en chaîne par polymérase en temps réel utilisant une sonde fluorescente et un couple d'amorces spécifiques d'Escherichia coli, de Staphylococcus epidermidis et de Klebsiella oxytoca.

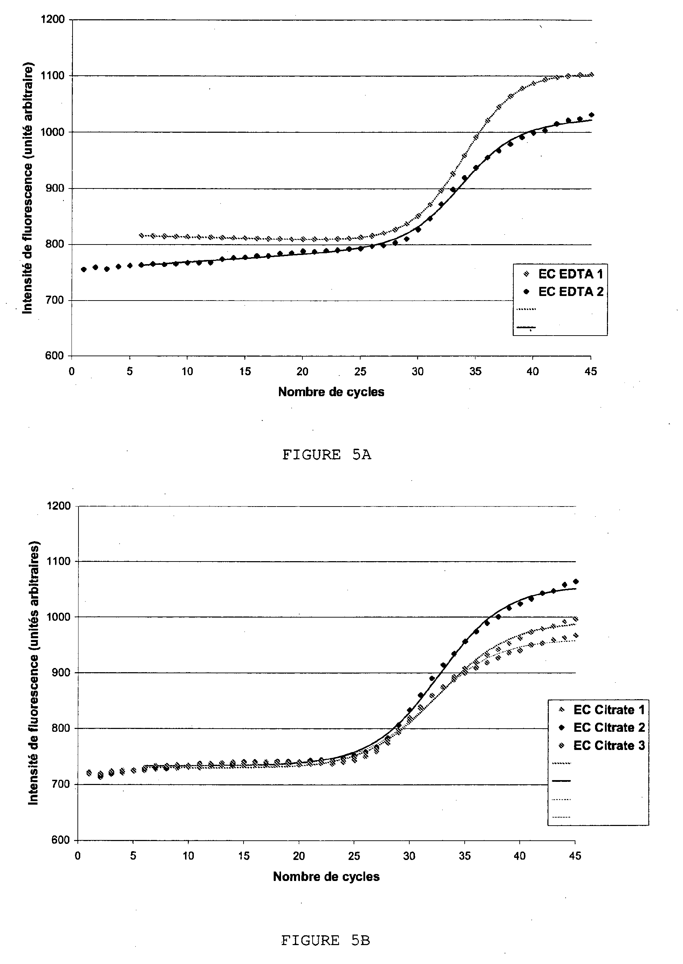

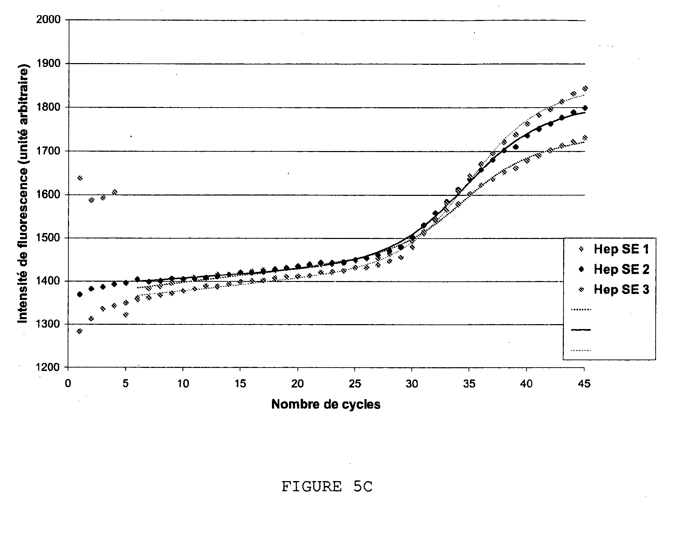

- la figure 5 illustre sous la forme de courbes l'identification et la quantification d'Escherichia coli présents dans des échantillons sanguins de sang total prélevé sur EDTA (figure 5A) et sur citrate de sodium (figure 5B) et de Staphylococcus epidermidis présents dans des échantillons sanguins de sang total prélevé sur héparine (figure 5C). La figure 5 illustre sous forme de courbe les résultats des réactions en chaîne par polymérase en temps réel utilisant une sonde fluorescente et un couple d'amorces spécifiques d'Escherichia coli et de Staphylococcus epidermidis.

### EXEMPLES

### I. Exemple I : Procédé de préparation d'échantillons sanguins

### 1.1 Procédé de préparation d'échantillons sanguins d'hémocultures

100 µl d'échantillon sanguin d'hémoculture ont été prélevés à l'aide d'une seringue stérile au travers du septum de flacons d'hémocultures. 1 mL d'eau osmosée filtrée (à l'aide d'un filtre dont les pores présentent un diamètre d'environ 0,22 µm) ont ensuite été ajoutés dans chaque échantillon.

Chaque échantillon a été filtré au travers d'une membrane de filtration en fluoride de polyvinylidène de diamètre de 25 à 32 mm et dont les pores présentent un diamètre allant de 0,2 µm à 1 µm. La membrane de filtration était contenue dans un support de filtre tel que décrit dans la demande de brevet US 2004/0208796.

Chaque membrane de filtration a ensuite été lavée avec 1 à 10 ml d'eau pure ou osmosée, par filtration à l'aide d'un filtre dont les pores présentent un diamètre d'environ 0,22 µm.

Après filtration, chaque membrane de filtration a été récupérée au moyen de pinces stériles et insérée dans un microtube stérile contenant de 200 µl à 1 ml:
- d'eau pure pour biologie moléculaire telle que celle commercialisée par Eppendorf, ou
- d'eau pure pour biologie moléculaire additionnée avec de l'albumine sérique bovine (BSA) à 0,7 %, ou
- d'eau pure pour biologie moléculaire additionnée avec du Tris HCl à 125 mM, de la Bétaïne à 160 mM et du dimethylsulfoxide (DMSO) à 5 %, à un pH de 9,3 ajusté par du NaOH à 1 M.

### I.2 Procédé de préparation d'échantillons sanguins de sang total

5 ml de sang total prélevés sur anticoagulant (EDTA, Héparine ou citrate de sodium) ont été ajoutés à 20 ml de solution d'agglutination des globules rouges.

Ladite solution d'agglutination comprenant la lectine *lens culinaris* à 25 µg/ml, du polyéthylène glycol (PEG) à 1% dans un milieu contenant 75% de bouillon de coeur de cervelle et 25% de Tryptone Soy Broth (TSB).

Après incubation 30 minutes à température ambiante, les globules rouges ont agglutiné en culot.

15 à 20 mL de surnageant du culot de globules rouges ont été prélevés et incubés en présence de 1 ml de solution d'agrégation plaquettaire.

Ladite solution d'agrégation comprenant l'anticorps monoclonal anti-CD9 à 45 µg/mL dans un milieu contenant 75% de bouillon de coeur de cervelle et 25% de TSB.
Après agrégation des plaquettes, la préparation a été filtrée au travers d'un filtre dont les pores présentent un diamètre d'environ 17 µm.

Cette étape de filtration a permis de retenir sur le filtre les agrégats plaquettaires et les cellules sanguines de taille supérieure à la taille des pores de du filtre.

Le filtrat a ensuite été à nouveau filtré au travers d'une membrane de filtration en fluoride de polyvinylidène de diamètre de 25 à 32 mm et dont les pores ont un diamètre allant de 0,2 µm à 1 µm. La membrane de filtration était contenue dans un support de filtre tel que décrit dans la Demande de Brevet US 2004/0208796.

La membrane de filtration a ensuite été lavée avec 8 à 20 ml d'eau pure ou osmosée, par filtration.

Après filtration, la membrane de filtration a été récupérée au moyen de pinces stériles et insérée dans un microtube stérile contenant entre 200 µl et 1 ml soit :
- d'eau pure pour biologie moléculaire telle que celle commercialisée par Eppendorf, soit
- d'eau pure pour biologie moléculaire additionnée avec de l'albumine sérique bovine (BSA) à 0,7 %, soit
- d'eau pure pour biologie moléculaire additionnée avec du Tris HCl à 125 mM, de la Bétaïne à 160 mM et du dimethylsulfoxide (DMSO) à 5 %, à un pH de 9,3 ajusté par du NaOH à 1 M.

### II. Exemple II : Identification de microorganismes présents dans des échantillons sanguins

### II.1 Procédé d'extraction d'ADN

L'extraction d'ADN a été réalisée à partir des membranes de filtration contenues dans des microtubes stériles, obtenues après la mise en oeuvre des procédés décrits dans l'exemple 1.

Chaque microtube a été soumis à une succession d'étapes de chauffage et/ou sonication, et/ou congélation telles que décrites dans le Maniatis (Sambrook, J., Fritsch, E.F. and Maniatis, T. in « Molecular cloning » (1992), Cold Spring Harbor Laboratory Press, Cold Spring Harbor, New York).

### II.2 Techniques de Réaction en chaîne par polymérase (PCR) utilisées

### II.2.1 Techniques de Réaction en chaîne par polymérase (PCR) quantitative en temps réel

30 à 42 µL d'ADN extraits selon le procédé décrit dans le paragraphe précédent (II.1) ont été ajoutés à 30 µL de mélange contenant la polymérase Taq® et des deoxyribonucleotides triphosphate (dNTP) en milieu tamponné.

12 µL de cette préparation ont été déposés dans des puits contenant la sonde fluorescente et le couple d'amorces spécifiques des souches de microorganismes à détecter tels que ceux décrits dans la Demande PCT WO 2004/024944. Le témoin négatif correspond à un puit dépourvu de sonde fluorescente.

Le témoin positif correspond à un puit comprenant une séquence synthétique d'ADN avec les sondes fluorescentes et le couple d'amorces spécifiques de ladite séquence.

Les résultats ont été exprimés d'une part sous la forme de courbes d'amplification montrant la variation d'intensité de la fluorescence en fonction des cycles d'amplification (courbes présentées sur les figures 1, 2 et 4).

D'autre part, les tableaux des figures 1 et 3 présentent pour chaque réaction en chaîne par polymérase réalisée :
- le cycle seuil ou Ct, correspondant au nombre de cycles minimal nécessaire pour atteindre la phase exponentielle d'amplification de l'ADN.
- l'amplitude correspondant à la différence entre l'intensité de fluorescence maximale et minimale obtenue.

### II.2.2 Techniques de Réaction en chaîne par polymérase (PCR) classique, puis migration sur gel d'agarose 7 µl d'ADN extraits selon le procédé décrit dans le paragraphe précédant (II.1) ont été ajoutés à 43 µL d'un mélange contenant de la polymérase Taq®, des deoxyribonucleotides triphosphate (dNTP) et le couple d'amorces spécifique des microorganismes à détecter, en milieu tamponné.

40 cycles d'amplification ont été réalisés.

Puis, 15 µL d'ADN amplifiés ont été pipetés, 3 µL de bleu de bromophénol y ont été ajoutés et cette préparation a été déposée dans un puit au sein d'un gel d'agarose.

Un courant a été appliqué afin de faire migrer les fragments d'ADN amplifiés en fonction de leur poids moléculaire (PM).

Une photo d'un gel d'agarose après migration est présentée sur la figure 3A.

### II.3 Identification d'Escherichia coli et de Staphylococcus epidermis présents dans des échantillons sanguins de sang total

5 ml de sang total ont été traités selon le protocole décrit dans le paragraphe I.2 dans lequel la membrane de filtration était en fluoride de polyvinylidène de 25 mm de diamètre et dont les pores présentent un diamètre d'environ 0,65 µm. 8mL d'eau osmosée filtrée à l'aide d'un filtre dont les pores présentent un diamètre d'environ 0,22 µm ont été utilisés pour laver la membrane de filtration.

Après extraction de l'ADN selon le protocole décrit dans le paragraphe II.1, des réactions en chaîne par polymérase (PCR) en temps réel ont été réalisées selon le protocole décrit dans le paragraphe II.2.1, en utilisant une sonde fluorescente et un couple d'amorces spécifiques d'Escherichia coli et de Staphylococcus epidermis.

La figure 1 (A et B) montre les résultats des réactions en chaîne par polymérase utilisant des sondes fluorescentes et un couple d'amorces spécifiques d'Escherichia coli.

La figure 2 (A et B) montre les résultats des réactions en chaîne par polymérase utilisant des sondes fluorescentes et un couple d'amorces spécifiques de Staphylococcus epidermis.

Ces résultats montrent que le procédé de préparation d'échantillon sanguin de sang total selon l'invention permet d'obtenir des échantillons dans lesquels les microorganismes peuvent être identifiés et quantifier de manière spécifique et reproductible par des techniques de réaction en chaîne par polymérase.

### II.4 Identification d'Escherichia coli présents dans des échantillons sanguins de sang total

5 ml de sang total ont été traités selon le protocole décrit dans le paragraphe I.2 dans lequel la membrane de filtration était en fluoride de polyvinylidène de 25 mm de diamètre et dont les pores présentent un diamètre d'environ 0,65 µm. 8mL d'eau osmosée filtrée à l'aide d'un filtre dont les pores présentent un diamètre d'environ 0 ,22 µm, ont été utilisés pour laver la membrane de filtration.

Après extraction de l'ADN selon le protocole décrit dans le paragraphe II.1, des dilutions de l'extrait d'ADN en eau pure pour biologie moléculaire ont été réalisées pour tester la sensibilité des PCR : dilution de l'extrait d'ADN de 1/100 à 1/50000.

Des réactions en chaîne par polymérase (PCR) quantitatives en temps réel selon le protocole décrit dans le paragraphe II.2.1 et en point final selon le protocole décrit dans le paragraphe II.2.2 ont ensuite été réalisées. Une sonde fluorescente et un couple d'amorces spécifiques d'Escherichia coli ont été utilisées.

La figure 3A montre une photo d'un gel d'agarose après réaction en chaîne par polymérase (PCR) classique et migration en utilisant une sonde fluorescente et un couple d'amorces spécifiques d'Escherichia coli.

La figure 3B montre les résultats des réactions en chaîne par polymérase (PCR) quantitatives en temps réel en utilisant des sondes fluorescentes et un couple d'amorces spécifiques d'Escherichia coli.

Ces résultats montrent que le procédé de préparation d'échantillon sanguin issu de sang total selon l'invention permet d'obtenir des échantillons dans lesquels les microorganismes peuvent être identifiés et quantifiés de manière spécifique, reproductible et avec une certaine sensibilité par des techniques de réaction en chaîne par polymérase en temps réel ou en point final.

### II.5 Identification d'Escherichia coli, de Staphylococcus epidermidis et de Klebsiella oxytoca présents dans des échantillons sanguins d'hémoculture

Des échantillons sanguins d'hémoculture ont été préparés selon le protocole décrit dans le paragraphe I.1 dans lequel la membrane de filtration était en fluoride de polyvinylidène de 25 mm de diamètre et dont le diamètre des pores est d'environ 0,65 µm. 3mL d'eau osmosée ont été utilisés pour laver la membrane de filtration.

Après extraction de l'ADN selon le protocole décrit dans le paragraphe II.1, des réactions en chaîne par polymérase (PCR) quantitatives en temps réel ont été réalisées selon le protocole décrit dans le paragraphe II.2.1, en utilisant une sonde fluorescente et un couple d'amorces spécifiques d'Escherichia coli, de Staphylococcus epidermidis et de Klebsiella oxytoca. Les résultats sont présentés sur la figure 4.

Ces résultats montrent que le procédé de préparation d'échantillon sanguin issu d'hémoculture selon l'invention permet d'obtenir des échantillons dans lesquels les microorganismes peuvent être identifiés et quantifiés de manière spécifique et reproductible par des techniques de réaction en chaîne par polymérase.

### II.6 Identification d'Escherichia coli et de Staphylococcus epidermis présents dans des échantillons sanguins de sang total prélevé sur différents anticoagulants

5 ml de sang total prélevé dans des tubes contenant un anticoagulant sanguin de type EDTA tripotassique, citrate de sodium ou héparine (héparine de lithium) ont été traités selon le protocole décrit dans le paragraphe I.2 dans lequel la membrane de filtration était en fluoride de polyvinylidène de 25 mm de diamètre et dont les pores présentent un diamètre d'environ 0,65 µm. 8mL d'eau osmosée filtrée à l'aide d'un filtre dont les pores présentent un diamètre d'environ 0,22 µm ont été utilisés pour laver la membrane de filtration.

Après extraction de l'ADN selon le protocole décrit dans le paragraphe II.1, des réactions en chaîne par polymérase (PCR) en temps réel ont été réalisées selon le protocole décrit dans le paragraphe II.2.1, en utilisant une sonde fluorescente et un couple d'amorces spécifiques d'Escherichia coli et de Staphylococcus epidermis.

Les figures 5 A et 5 B montre les résultats des réactions en chaîne par polymérase utilisant des sondes fluorescentes et un couple d'amorces spécifiques d'Escherichia coli pour de l'ADN bactérien issu de sang total prélevé sur EDTA tripotassique (figure 5A) et sur citrate de sodium (figure 5B).

La figure 5 C montre les résultats des réactions en chaîne par polymérase utilisant des sondes fluorescentes et un couple d'amorces spécifiques de Staphylococcus epidermis pour de l'ADN bactérien issu de sang total prélevé sur héparine (figure 5C).

Ces résultats montrent que le procédé de préparation d'échantillon sanguin issu de sang total prélevé sur différents anticoagulants selon l'invention permet d'obtenir des échantillons dans lesquels les microorganismes peuvent être identifiés et quantifiés de manière spécifique et reproductible par des techniques de réaction en chaîne par polymérase.

## Revendications

1. Procédé d'extraction d'ADN de microorganismes présents dans un échantillon sanguin comprenant les étapes suivantes :
i) la filtration d'un échantillon sanguin au travers d'une membrane de filtration dont les pores présentent un diamètre allant de 0,01 µm à 50 µm, en particulier de 0,1 µm à 10 µm et tout particulièrement de 0,2 µm à 1 µm ;
ii) le lavage de ladite membrane de filtration, dans lequel ledit lavage lyse les globules rouges de l'échantillon sanguin ; et
iii) l'extraction des acides désoxyribonucléiques des microorganismes éventuellement présents sur ladite membrane de filtration.

2. Procédé selon la revendication 1, **caractérisé en ce qu'**il comprend en outre l'étape suivante :
iv) l'identification des microorganismes notamment des bactéries, des virus, des protozoaires et/ou des champignons présents dans ledit échantillon sanguin.

3. Procédé selon la revendication 2, **caractérisé en ce que** l'étape iv) comprend l'utilisation d'une technique de biologie moléculaire utilisant une activité de type polymérase choisie dans le groupe comprenant la réaction en chaîne par polymérase en point final, la réaction en chaîne par polymérase en temps réel, la réaction en chaîne par polymérase multiplex, la réaction en chaîne par polymérase qualitative, la réaction en chaîne par polymérase semi-quantitative et la réaction en chaîne par polymérase quantitative.

4. Procédé selon l'une quelconque des revendications 1 à 3, **caractérisé en ce qu'**il comprend en outre l'étape suivante :
v) l'identification d'au moins un gène de résistance à un antibiotique chez au moins un microorganisme présent dans ledit échantillon sanguin.

5. Procédé selon la revendication 4, **caractérisé en ce que** l'étape v) comprend l'utilisation d'une technique de réaction en chaîne par polymérase.

6. Procédé selon l'une quelconque des revendications 1 à 5, **caractérisé en ce qu'**il comprend en outre l'étape suivante :
vi) la détermination de la quantité de microorganismes notamment des bactéries, des virus, des protozoaires et/ou des champignons présents dans ledit échantillon sanguin.

7. Procédé selon l'une quelconque des revendications 1 à 6, **caractérisé en ce que** ladite membrane de filtration est choisie dans le groupe comprenant les membranes en fluoride de polyvinylidène, en polyester, en nylon, en polypropylène, en polycarbonate et en polyethersulfone, en particulier en fluoride de polyvinylidène.

8. Procédé selon l'une quelconque des revendications 1 à 7, **caractérisé en ce que** ladite membrane de filtration n'est pas à base de cellulose.

9. Procédé selon l'une quelconque des revendications 1 à 8, **caractérisé en ce que** ledit échantillon sanguin est choisi dans le groupe comprenant :
- un échantillon sanguin de sang total ; et
- un échantillon sanguin d'hémoculture.

10. Procédé selon l'une quelconque des revendications 1 à 9, **caractérisé en ce qu'**il comprend préalablement à l'étape i), les étapes suivantes :
a) l'addition au sang total ou à l'hémoculture d'une solution d'agglutination des globules rouges et/ou d'une solution d'agrégation plaquettaire ; et
b) la filtration de la préparation obtenue à l'étape a) au travers d'un filtre dont les pores présentent un diamètre allant de 2 µM à 50 µM, en particulier allant de 10 µM à 25 µM et tout particulièrement de 17 µm.

11. Procédé selon la revendication 10, **caractérisé en ce que** ladite solution d'agglutination comprend au moins un agent d'agglutination choisi dans le groupe comprenant les lectines, le polyéthylènimine, la polyvinylpyrrolidone, les gélatines, les dextrans et les polyethylènes glycols, en particulier les lectines de *Lens culinaris.*

12. Procédé selon l'une des revendications 10 ou 11, **caractérisé en ce que** ladite solution d'agrégation plaquettaire comprend au moins un agent d'agrégation plaquettaire choisi dans le groupe comprenant par les anticorps spécifiques d'un antigène plaquettaire, la thrombine, la trypsine, le collagène, le thromboxane A2, le facteur d'activation plaquettaire, l'adrénaline, l'acide arachidonique, la sérotonine et l'épinéphrine, en particulier les anticorps spécifiques d'un antigène plaquettaire et le collagène.

## Patentansprüche

1. Verfahren zur Extraktion von RNA aus in einer Blutprobe vorhandenen Mikroorganismen mit folgenden Schritten :
i) das Filtrieren einer Blutprobe durch einen Membranfilter dessen Poren einen Durchmesser von 0,01 µm bis 50 µm, besonders von 0,1 µm bis 10 µm und ganz besonders von 0,2 µm bis 1 µm aufweisen;
ii) das Waschen des Membranfilters, in welchem das Waschen die roten Blutkörperchen der Blutprobe lysiert ; und
iii) die Extraktion der Desoxyribonukleinsäuren der möglicherweise auf dem Membranfilter vorhandenen Mikroorganismen.

2. Verfahren nach Anspruch 1, **dadurch gekennzeichnet, dass** es ferner folgenden Schritt umfasst :
iv) die Identifizierung der in der Blutprobe vorhandenen Mikroorganismen, insbesondere der Bakterien, Viren, Protozoen und/oder der Pilze.

3. Verfahren nach Anspruch 2, **dadurch gekennzeichnet, dass** der Schritt iv) die Verwendung einer Technik aus der Molekularbiologie umfasst, die eine Aktivität des Typs Polymerase verwendet, ausgewählt aus der Gruppe umfassend Endpunkt-Polymerase-Kettenreaktion, Echtzeit-Polymerase-Kettenreaktion, Multiplex-Polymerase-Kettenreaktion, qualitative Polymerase-Kettenreaktion, halbquantitative Polymerase-Kettenreaktion und quantitative Polymerase-Kettenreaktion.

4. Verfahren nach einem der Ansprüche 1 bis 3, **dadurch gekennzeichnet, dass** es ferner folgenden Schritt umfasst :
v) das Identifizieren mindestens eines gegen ein Antibiotikum resistenten Gens bei mindestens einem in der Blutprobe vorhandenen Mikroorganismus.

5. Verfahren nach Anspruch 4, **dadurch gekennzeichnet, dass** der Schritt v) die Verwendung einer Polymerase-Kettenreaktionstechnik umfasst.

6. Verfahren nach einem der Ansprüche 1 bis 5, **dadurch gekennzeichnet, dass** es ferner folgenden Schritt umfasst:
vi) die Bestimmung der Menge der Mikroorganismen, besonders der in der Blutprobe vorhandenen Bakterien, Viren, Protozoen und/oder Pilze.

7. Verfahren nach einem der Ansprüche 1 bis 6, **dadurch gekennzeichnet, dass** der Membranfilter aus der Gruppe ausgewählt wird, die Membranen aus Polyvinylidenfluorid, aus Polyester, aus Nylon, aus Polypropylen, aus Polycarbonat und aus Polyethersulfon, insbesondere aus Polyvinylidenfluorid umfasst.

8. Verfahren nach einem der Ansprüche 1 bis 7, **dadurch gekennzeichnet, dass** der Membranfilter nicht auf Zellulosebasis besteht.

9. Verfahren nach einem der Ansprüche 1 bis 8, **dadurch gekennzeichnet, dass** die Blutprobe aus der Gruppe ausgewählt wird, welche die folgenden umfasst :
- eine Vollblutprobe ; und
- eine Hämokultur-Blutprobe.

10. Verfahren nach einem der Ansprüche 1 bis 9, **dadurch gekennzeichnet, dass** es vor dem Schritt i), die folgenden Schritte umfasst :
a) das Hinzufügen einer Lösung zur Agglutination der roten Blutkörperchen und/oder einer Lösung zur Blutplättchenaggregation zu dem Vollblut oder der Hämokultur; und
b) das Filtrieren des in Schritt a) erhaltenen Gemischs durch einen Filter, dessen Poren einen Durchmesser von 2 µm bis 50 µm, besonders von 10 µm bis 25 µm und ganz besonders von 17 µm aufweisen.

11. Verfahren nach Anspruch 10, **dadurch gekennzeichnet, dass** die Agglutinationslösung mindestens ein Agglutinationsmittel enthält, das aus der Gruppe ausgewählt wird, die Lektine, Polyethylenimine, Polyvinylpyrrolidon, Gelatine, Dextrane und Polyethylenglycole, insbesondere die Lektine der *Lens culinaris* umfasst.

12. Verfahren nach einem der Ansprüche 10 oder 11, **dadurch gekennzeichnet, dass** die Blutplättchen-Aggregationslösung mindestens ein Blutplättchen-Aggregationsmittel umfasst, das aus der Gruppe ausgewählt wird, die durch die spezifischen Antikörper eines Blutplättchen-Antigens Thrombin, Trypsin, Kollagen, Thromboxan A2, den Blutplättchenaktivierungsfaktor, Adrenalin, Arachidonsäure, Serotonin und Epinephrin, insbesondere die spezifischen Antikörper eines Blutplättchen-Antigens und Kollagen umfasst.

## Claims

1. Method of extracting DNA from microorganisms present in a blood sample comprising the following steps:
i) filtration of a blood sample through a filtration membrane whose pores have a diameter ranging from 0.01 µm to 50 µm; in particular from 0.1 µm to 10 µm and more particularly from 0.2 µm to 1 µm;
ii) washing of said filtration membrane, wherein said washing lyses the red blood cells of the blood sample; and
iii) extraction of the deoxyribonucleic acids from the microorganisms present on said filtration membrane.

2. Method according to claim 1, **characterised in that** it also comprises the following step:
iv) identification of the microorganisms in particular bacteria, viruses, protozoa and/or fungi present in said blood sample.

3. Method according to claim 2, **characterised in that** step iv) comprises the use of molecular biology technique using a polymerase type activity chosen from the group comprising end point polymerase chain reaction, real-time polymerase chain reaction, multiplex polymerase chain reaction, qualitative polymerase chain reaction, semi quantitative polymerase chain reaction and quantitative polymerase chain reaction.

4. Method according to any one of claims 1 to 3, **characterised in that** it also comprises the following step:
v) identification of at least one antibiotic resistance gene in at least one microorganism possibly present in said blood sample.

5. Method according to claim 4, **characterised in that** step v) comprises the use of a polymerase chain reaction technique.

6. Method according to anyone of claims 1 to 5, **characterised in that** it also comprises the following step:
vi) the determination of the quantity of microorganisms, in particular bacteria, viruses, protozoa and/or fungi present in said blood sample.

7. Method according to anyone of claims 1 to 6, **characterised in that** said filtration membrane is chosen from the group comprising membranes made from polyvinylidene fluoride, polyester, nylon, polypropylene, polycarbonate and polyethersulfone, in particular polyvinylidene fluoride.

8. Method according to anyone of claims 1 to 7, **characterised in that** said filtration membrane is not based on cellulose.

9. Method according to anyone of claims 1 to 8, **characterised in that** said blood sample is chosen from the group comprising:
- a whole blood sample; and
- a haemoculture blood sample.

10. Method according to anyone of claims 1 to 9, **characterised in that** it comprises, prior to step i), the following steps:
a) addition to the whole blood or to the haemoculture of a red blood cells agglutination solution and/or a platelet aggregation solution; and
b) filtration of the preparation obtained at step a) through a filter whose pores have a diameter ranging from 2 µm to 50 µm, in particular from 10 µm to 25 µm and more particularly of 17 µm.

11. Method according to claim 10, **characterised in that** said agglutination solution comprises at least one agglutination agent chosen from the group comprising lectins, polyethylenimine, polyvinylpyrrolidone, gelatines, dextrans and polyethylene glycols, in particular lectins of *Lens culinaris*

12. Method according to one of claims 10 or 11, **characterised in that** said platelet aggregation solution comprises at least one platelet aggregation agent chosen from the group comprising specific antibodies of a platelet antigen, thrombin, trypsin, collagen, thromboxane A2, the platelet activation factor, adrenalin, arachidonic acid, serotonin and epinephrine, in particular specific antibodies of a platelet antigen and collagen.
